# EUROPÄISCHE PATENTSCHRIFT

(11) **EP 1 069 881 B1**
(45) Veröffentlichungstag und Bekanntmachung des Hinweises auf die Patenterteilung: **27.08.2003**
(21) Anmeldenummer: 99958053.3
(22) Anmeldetag: 16.11.1999
(51) Int. Cl.: A61H 31/00, A61M 16/00

(54) **VORRICHTUNG ZUR WIEDERBELEBUNG VON PATIENTEN MIT HERZSTILLSTAND**
DEVICE FOR REANIMATING PATIENTS SUFFERING FROM CARDIAC ARREST
DISPOSITIF POUR REANIMER DES PATIENTS VICTIMES D'ARRET CARDIAQUE

(30) Priorität: 12.12.1998 DE 19857421
(43) Veröffentlichungstag der Anmeldung: 24.01.2001
(73) Patentinhaber: Sessler, Stefan, 72474 Winterlingen (DE)
(72) Erfinder: Sessler, Stefan, 72474 Winterlingen (DE)
(74) Vertreter: Möbus, Daniela, Dr.-Ing.
(86) Internationale Anmeldenummer: EP9908810
(87) Internationale Veröffentlichungsnummer: WO00035404

(56) Entgegenhaltungen:
- DE-A- 2 521 121
- FR-A- 1 331 573
- FR-A- 2 382 889
- US-A- 3 425 409
- US-A- 3 489 140
- US-A- 3 512 522
- US-A- 5 257 619
- US-A- 5 327 887

## Beschreibung

Die Erfindung betrifft eine Vorrichtung zur Wiederbelebung von Patienten mit Herzstillstand mti den Merkmalen des Oberbegriffs des Anspruchs 1.

Die Reanimationserfolge nach einem Herzstillstand sind nach wie vor gering. Nach einer Statistik überleben weniger als 5 % der Patienten längerfristig nach einer außerklinischen Reanimation. Längerfristige Reanimationserfolge im Krankenhaus liegen bei 10 %. Ein großes Problem bei der Reanimation ist, dass große Bevölkerungsteile die Technik der Wiederbelebung nur unzureichend oder gar nicht beherrschen. Sogar bei ehrenamtlichen Mitgliedern in Hilfsorganisationen wie DRK oder DLRG besteht oft eine sehr große Unsicherheit bei der Ausübung der Reanimationsmaßnahmen. Aus dieser Situation heraus sind in der Vergangenheit verschiedene Reanimationsgeräte der eingangs genannten Art entwickelt und auf den Markt gebracht worden. Darunter befinden sich Geräte, die mit einem Spanngurt am Patienten befestigt werden oder Geräte, die am Patienten überhaupt nicht fixiert werden oder Geräte, die für die Fixierung des Gerätes ein Unterlegbrett aufweisen. Allen diesen Reanimationsgeräten ist gemeinsam, dass sie entweder nur sehr schlecht transportiert werden können, weil sie sehr groß oder sehr schwer sind, oder dass sie eine externe Energieversorgung aufweisen, weshalb sie unter Umständen nicht überall einsetzbar sind.

Die bisher bekannten Reanimationsgeräte arbeiten entweder nach dem CPR-Verfahren oder nach dem ACD-Verfahren.

Das CPR-Verfahren stellt die Standard-Reanimation dar. Hierbei unterscheidet man zwischen der Einhelfer- und der Zweihelfer-Methode. Bei der Einhelfer-Methode werden fünfzehn Herzdruckmassagen ausgeübt, worauf zwei Beatmungszüge folgen. Bei der Zweihelfer-Methode werden fünf Herzdruckmassagen ausgeführt, worauf ein Beatmungszug folgt.

Bei der ACD-Methode wird nach der Kompression des Brustkorbs dieser mit einer Saugglocke wieder angehoben. Dieses aktive Dekomprimieren fördert den venösen Rückfluss des Blutes, was die Effektivität der Herz-Lungen-Wiederbelebung verbessert.

Aus der US-A 3,489,140 ist ein Reanimationsgerät mit einem als Portal ausgebildeten Träger für eine Hubvorrichtung für einen auf den Brustkorb absenkbaren Stempel bekannt, bei dem das Portal in Querträger und Beine zerlegbar und die Portalbreite an den Brustkorb des Patienten anpassbar ist. Dazu weisen die Beine obere Einsätze mit seitlichen Löchern auf, die entlang des schienenartigen Querträgers bewegbar sind. Der Querträger weist seitliche Lochreihen auf, die mit den Löchern der Einsätze fluchten. Die Löcher der Einsätze oder des Querträgers sind mit einem Gewinde versehen, sodass eine Veränderung der Breite des Portals durch Verschieben der Beineinsätze im Querträger und Fixieren der gewünschten Position durch Schrauben, die durch entsprechende Lochpaarungen zwischen Querträger und Einsätzen geführt und darin verschraubt sind, möglich ist. Diese Art der Breitenanpassung der Vorrichtung ist jedoch äußerst umständlich und im Notfall viel zu zeitraubend.

Die vorliegende Erfindung hat die Aufgabe, eine Vorrichtung zur Wiederbelebung von Patienten mit Herzstillstand dahin gehend zu verbessern, dass sie flexibel und einfach zu handhaben ist.

Die Erfindung löst die gestellte Aufgabe mittels einer Vorrichtung mit den Merkmalen des Anspruchs 1. Durch diesen Aufbau ist das Reanimationsgerät leicht an Patienten mit unterschiedlicher Brustkorbgröße anbringbar und unabhängig von den örtlichen Gegebenheiten einsetzbar. Die Portalbauweise des Trägers ist außerdem sehr stabil, da damit eine symmetrische Abstützung der Vorrichtung beidseitig des Patienten möglich ist.

Die Vorrichtung weist einen Einstellgriff auf, mittels dessen das Portal mindestens in seiner Breite verstellt werden kann. Somit kann das Portal mit einem einzigen Griff sowohl in der Breite und, falls es auch höhenverstellbar ist, auch in der Höhe verstellt werden.

Um beim Beatmungsvorgang eine zu schnelle Zufuhr der Beatmungsluft zu verhindern, kann die auf den Stempel oder die Saugglocke wirkende Bedienkraft insbesondere mittels einer Feder und/oder einer Düse erhöht werden.

Als Widerlager für die Reanimationsvorrichtung beim Betätigen der Hubvorrichtung, können die Portalbeine mit nach innen zeigenden Füßen, die vorzugsweise keilförmig ausgebildet sind, versehen sein. Diese Füße werden unter den Brustkorb des Patienten geschoben und verhindern damit ein Anheben der gesamten Vorrichtung, wenn der Brustkorb mittels des Stempels oder der Saugglocke komprimiert wird.

Die Anpassung der Reanimationsvorrichtung an unterschiedliche Breiten bei verschiedenen Brustkörben kann beispielsweise dadurch erfolgen, dass der Querträger des Portals als Teleskoprohr ausgebildet ist, an dem die Beine ausziehbar angeordnet sind. Dabei kann im Teleskoprohr und/oder in den Beinen zum automatischen Ausmitteln der Hubvorrichtung über dem Patienten ein Federmechanismus oder ein Zahnrad-Zahnstangenmechanismus angeordnet sein.

Alternativ zur Ausbildung des Querträgers als ein Teleskoprohr können der linke und der rechte Querträger des Portals anstatt in einer Linie zu fluchten versetzt zueinander angeordnet sein. Somit ist ebenfalls eine Verstellung der Breite des Portals möglich. Außerdem kann durch die versetzte Anordnung des linken und rechten Querträgers die Vorrichtung zum Transport in ihren äußeren Abmessungen so stark reduziert werden, dass sie nicht zerlegt werden muss.

Bei der versetzten Anordnung des linken und rechten Querträgers kann die Hubvorrichtung mittels eines an den Querträger montierten Seil- oder Kettenzugs vorteilhafterweise über dem Patienten ausgemittelt werden.

Um die Reanimationsvorrichtung auf die jeweilige Höhe des Brustkorbs bei unterschiedlichen Patienten individuell einstellen zu können, kann die Hubvorrichtung höhenverstellbar am Portal angeordnet sein. Auch eine Höhenverstellung der Beine des Trägers wäre möglich.

Damit die Reanimationsvorrichtung von einer externen Energieversorgung unabhängig ist und dennoch eine Krafterleichterung für den Helfer gegeben ist, kann ein Übersetzungsmechanismus verwendet werden. Dieser kann als Hebel oder Getriebe, hydraulischer, pneumatischer Druckübersetzer oder dergleichen umgesetzt werden. Nach der Betätigung kann der Mechanismus selbständig in seine Grundstellung zurückfahren.

Die Hubvorrichtung kann außerdem einen Hubzylinder aufweisen, der aus der Umgebung Luft ansaugt, die zur künstlichen Beatmung des Patienten über eine Maske oder einen Tubus an den Patienten abgebbar ist. Auf diese Weise ist die Reanimationsvorrichtung auch zur Beatmung des Patienten und unabhängig von Sauerstoffflaschen einsetzbar. Damit sowohl eine Maske wie auch ein Tubus eingesetzt werden kann, kann die Reanimationsvorrichtung einen Adapter aufweisen.

Damit die Luft nicht während einer Hubbewegung nach unten zur Ansaugöffnung aus dem Zylinder austreten kann, kann die Hubvorrichtung ein Rückschlagventil aufweisen, sodass die Luft nur in Richtung der Gesichtsmaske oder des Tubus entweichen kann.

Zur Vermeidung von Bedienungsfehlern kann die Vorrichtung vorzugsweise automatisch zwischen Herzdruckmassage und Beatmung wechseln. Je nach Anwendungsfall kann die Vorrichtung dabei nach dem CPR-Verfahren und/oder dem ACD-Verfahren betrieben werden.

Für Patienten mit einem großen Brustkorb muss ein größerer Hubweg zurückgelegt werden als für Menschen mit einem kleineren Brustkorb. Außerdem ist das Beatmungsvolumen ebenso abhängig von der Brustkorbgröße. Aus diesem Grund kann der Hubweg für die Herzmassage als auch das Beatmungvolumen der Vorrichtung einstellbar sein.

Damit auch ungeübte Anwender der Reanimationsvorrichtung die Vorrichtung sofort sicher bedienen können, ohne auf die jeweilige Brustkorbgröße des Patienten achten zu müssen, kann sich sowohl der Hubweg für die Herzmassage als auch das durch den Hubzylinder abgegebene Beatmungsvolumen automatisch abhängig von der Höhenposition der Hubvorrichtung einstellen. Bedienungsfehler lassen sich durch diese Maßnahme praktisch ausschließen.

Um einen Hinweis auf den eingestellten Hubweg und das eingestellte Beatmungsvolumen zu erhalten, kann die Vorrichtung eine Skala für den Hubweg und das Beatmungsvolumen aufweisen.

Vorteilhafterweise kann die Reanimationsvorrichtung einen Sauerstoffanschluss für eine Sauerstoffflasche oder für ein dazwischengeschaltetes Sauerstoffreservoir aufweisen.

Aus Gründen des einfachen Transports kann die Vorrichtung zweckmäßigerweise zerlegbar sein.

Je nach gewünschtem Anwendungsfall kann die Vorrichtung auch als reines Beatmungsgerät oder als reines Herzmassagegerät verwendet werden.

Um auch nach längerem Nichtgebrauch stets einsatzbereit zu sein, kann die Reanimationsvorrichtung vorzugsweise wartungsfrei gestaltet sein.

Aus hygienischen Gründen kann die Vorrichtung außerdem dampfsterilisierbar sein.

Bei Einsatz der Vorrichtung nach der ACD-Methode kann die Saugglocke beim Rückhub über die Brustkorbhöhe hinausgefahren werden, um die erforderliche Dekompression zu erzeugen.

Um das Bedienpersonal zu entlasten, kann die Dekompressionskraft durch eine Feder freigesetzt werden, die beim Druckhub vorgespannt wird.

Bei schwächeren Federn kann die Dekompression mittels eines krafterleichternden Mechanismus, beispielsweise durch einen Hebel oder mittels externer Energie unterstützt werden.

Die Hubvorrichtung kann, wie bereits erwähnt, über einen Hebel bedient werden. Besonders vorteilhaft ist es, wenn dieser Hebel drehbar am Gerät gelagert ist, damit das Gerät von allen oder wenigstens von mehreren Seiten aus bedient werden kann. Der Hebel kann dabei in bestimmten Winkelpositionen einrastbar ausgestaltet werden. Eine solche Ausgestaltung der Vorrichtung hat auch bei der Bedienung der Vorrichtung durch Linkshänder Vorteile. Auch bei beengten Platzverhältnissen, beispielsweise im Mittelgang eines Flugzeugs, bietet ein solcher drehbar gelagerter Hebel Vorteile.

Weitere Vorteile lassen sich erzielen, wenn die Vorrichtung eine Umschaltmechanik aufweist, die automatisch vom Beatmungsbetrieb auf Herzdruckmassagebetrieb und umgekehrt wechselt. Eine solche Umschaltmechanik kann beispielsweise durch einen Verzahnungsmechanismus oder eine Führungsbahn realisiert werden.

Wie bereits erwähnt, lässt sich das Beatmungsvolumen automatisch abhängig von der Höhenposition der Hubvorrichtung einstellen. Es kann dabei auch eine zusätzliche Begrenzung in Form eines Anschlags vorgesehen werden, wodurch es möglich ist, unabhängig von der Brustkorbgröße ein konstantes Beatmungsvolumen zu erzeugen, wobei die Eindrücktiefe von Hand einstellbar bzw. automatisch einstellbar sein kann.

Das Gerät kann auch durch einen Defibrillator erweitert werden und damit ein Kombigerät für die Wiederbelebung bilden. Die ermittelten Daten aus der Diagnose eines Automatisch Externen Defibrillators (AED) können u. a. dazu verwendet werden, die Funktionen des Reanimationsgerätes zu starten oder zu beenden. Das Reanimationsgerät kann bei einer solchen Erweiterung ohne oder mit externer Energie arbeiten.

Nachfolgend wird ein Ausführungsbeispiel einer erfindungsgemäßen Vorrichtung zur Wiederbelebung von Patienten mit Herzstillstand anhand der beiliegenden Zeichnung näher erläutert.

Im Einzelnen zeigen:
- Fig. 1: eine Seitenansicht einer Reanimationsvorrichtung;
- Fig. 2: eine Draufsicht auf eine Reanimationsvorrichtung aus Fig. 1;
- Fig. 3: eine Stirnansicht auf eine weitere Ausführungsform einer Reanimationsvorrichtung,
- Fig. 4: eine Prinzipskizze zweier zueinander versetzter Querträger.

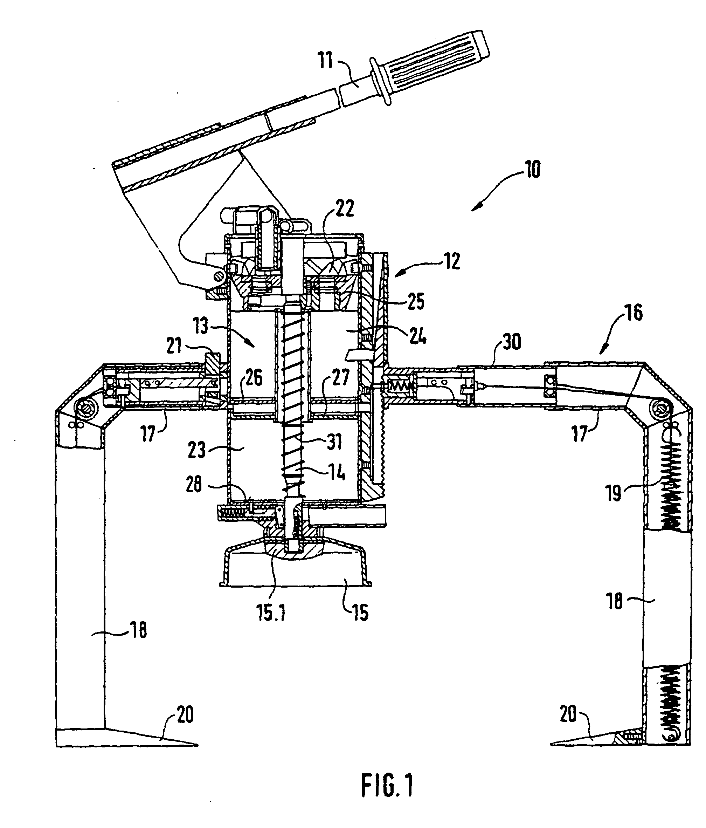

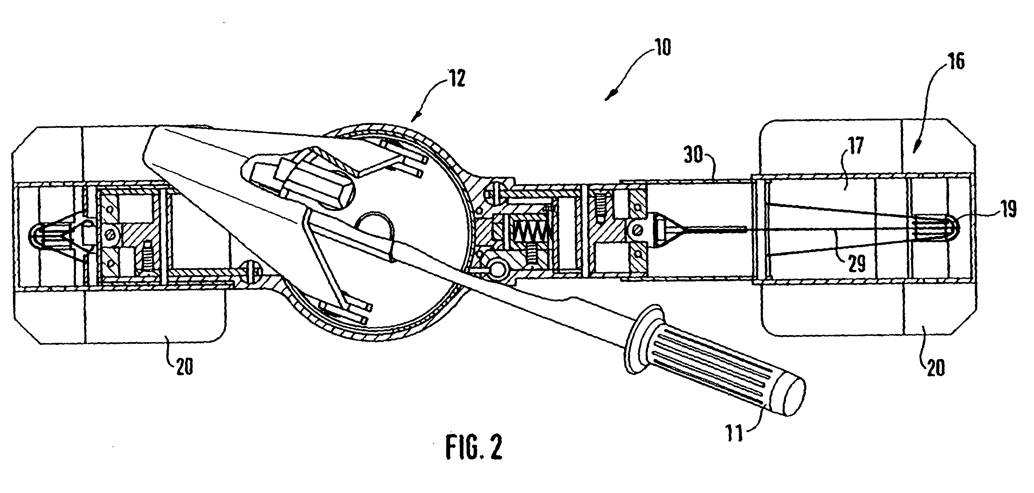

Fig. 1 zeigt eine Vorrichtung 10 zur Wiederbelebung von Patienten mit Herzstillstand. Mittels des Hebels 11 wird eine Hubvorrichtung 12, die einen Hubzylinder 13 aufweist, betätigt. Sie wird also rein mechanisch betätigt und ist dadurch von externer elektrischer, pneumatischer oder hydraulischer Energie unabhängig. Mittels einer Druckstange 14 versetzt die Hubvorrichtung 12 eine Saugglocke 15 mit Stempel 15.1 in eine periodische Auf- und Abbewegung. Der Stempel 15.1 sitzt im Anwendungsfall auf dem Brustkorb-Druckpunkt eines Patienten auf, wobei durch die Auf- und Abbewegung des Stempels 15.1 eine Herzdruckmassage des Patienten durchgeführt wird. Mit jedem Rückhub wird der Brustkorb mittels der Saugglocke 15 angehoben. Die Hubvorrichtung 12 ist auf einem Träger in Form eines Portal 16 angeordnet. Das Portal 16 weist einen Querträger 17 auf, in dem ein Teleskoprohr 30 steckt. Durch das Teleskoprohr 30 kann das Portal 16 auf die jeweilige Brustkorbbreite des zu behandelnden Patienten eingestellt werden. In den Portalbeinen 18 sind Federn 19 untergebracht, die über einen Seilzug 29 (Fig. 2) mit dem Teleskoprohr 30 gekoppelt sind. Da die Federn 19 in den Beinen 18 links und rechts identisch sind, wird beim Auseinanderziehen des Teleskoprohrs 30 die Hubvorrichtung 12 automatisch ausgemittelt. Dadurch befinden sich die Saugglocke 15 und der Stempel 15.1 immer direkt mittig zum Brustkorb des Patienten. Die Portalbeine 18 stehen auf nach innen zeigenden Füßen 20. Die Füße 20 sind keilförmig ausgebildet und können somit leicht unter den Brustkorb des Patienten geschoben werden. Während der Behandlung fixiert somit der Patient, dadurch dass er auf den Füßen 20 aufliegt, die Vorrichtung 10 durch sein Gewicht. Damit die Vorrichtung 10 in einem handlichen Transportkoffer transportiert werden kann, können die Füße 20 umgeklappt oder durch einfache Handgriffe von den Portalbeinen 18 gelöst werden und der Querträger 17 zusammen mit den Portalbeinen 18 durch einen Druck auf den Entriegelungsknopf 21 von der Hubvorrichtung 12 gelöst werden. Somit lässt sich die Vorrichtung 10 durch wenige Handgriffe rasch zerlegen und mühelos in den Transportkoffer verstauen.

Die Vorrichtung 10 ist sowohl zur Herzmassage als auch zur Beatmung des Patienten einsetzbar. Durch eine Rastscheibe 22 lässt sich das Verhältnis der Beatmungshübe zu den Massagehüben bestimmen. Der Hubzylinder 13 weist zwei Kammern 23 und 24 auf. Die Kammer 24 ist oben durch einen Drehkolben 25 und unten durch ein Trennblech 26 begrenzt. Die Kammer 23 ist oben durch eine Kolbenscheibe 27 und unten durch den Zylinderboden 28 begrenzt. Durch die Aufteilung des Zylinders 13 in die beiden Kammern 23 und 24 kann man mit einer kleineren Bewegung des Hebels 11 das gesamte Volumen des Zylinders 13 in die Lungen des Patienten pumpen.

Fig. 2 zeigt die Vorrichtung 10 aus Fig. 1 von oben. über den Hebel 11 wird die Hubvorrichtung 12 betätigt und dadurch die Herzdruckmassage durchgeführt oder der Patient mit Beatmungsluft versorgt. Innerhalb des Querträgers 17 des Portals 16 verläuft ein Seilzug 29, der die Feder 19 mit dem Teleskoprohr 30 verbindet. Das Portal 16 steht auf den breit ausgebildeten Füßen 20, die unter den Patientenkörper geschoben werden.

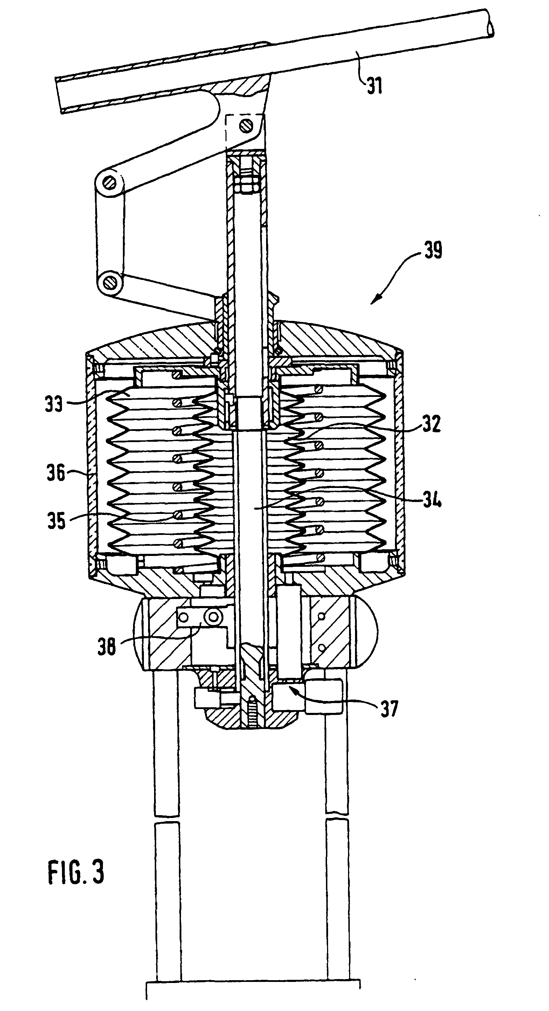

Fig. 3 zeigt eine Vorrichtung 39 zur Wiederbelebung von Patienten mit Herzstillstand. Mittels des Hebels 31 werden die das Beatmungsvolumen bestimmenden Faltenbälge 32 und 33 zusammengedrückt, wodurch die Beatmungsluft dem Patienten zugeführt wird. Der innere Faltenbalg 32 hat die Aufgabe, eine Druckstange 34 und die mit ihm verbundene Mechanik vom Beatmungsvolumen zu trennen. Der äußere Faltenbalg 33 grenzt das Beatmungsvolumen von der Außenumgebung ab. Im Inneren des äußeren Faltbalges 33 ist eine Feder 35 angeordnet, die die beiden an ihrem Boden und an ihrer Decke miteinander in Berührungskontakt stehenden Faltenbälge 32 und 33 nach dem ausgeführten Beatmungshub wieder auseinanderdrückt. Außerdem erhöht die Feder 35 die Bedienkraft beim Beatmen, um eine zu schnelle Zufuhr des Beatmungsvolumens zu verhindern. Eine Düse 37 dient ebenfalls zur Erhöhung der Bedienkraft während der Beatmung, indem sie den Querschnitt des Luftkanals, durch den die Luft dem Patienten zugeführt wird, verengt. Ein transparenter Schutzzylinder 36 kapselt den äußeren Faltenbalg 33 nach außen ab. Durch das transparente Material des Schutzzylinders 36 kann der Bediener der Vorrichtung 39 den Beatmungsvorgang beobachten. Ein Anschlagkeil 38 begrenzt den Hub der Druckstange 34.

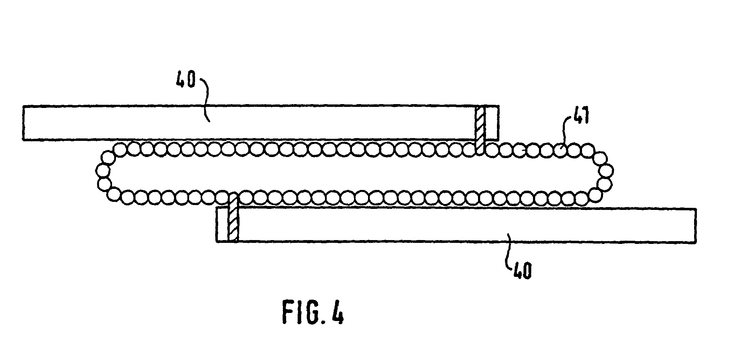

Fig. 4 zeigt die prinzipielle Anordnung der beiden zueinander versetzten Querträger 40. Diese sind in ihrer Längsrichtung verschiebbar. Die beiden Querträger 40 sind durch einen geschlossen umlaufenden Seilzug 41 miteinander verbunden. Durch den geschlossen umlaufenden Seilzug 41 werden die beiden Querträger gleichzeitig jeweils um denselben Abstand nach links oder nach rechts verschoben, wodurch die Hubvorrichtung beim Auseinanderziehen der beiden Querträger 40 genau über dem Patienten ausgemittelt wird. Vor dem Einsatz werden die Querträger 40 auseinandergezogen und nach dem Einsatz wieder zusammengeschoben. Somit kann die gesamte Vorrichtung in ihren Außenabmessungen so stark reduziert werden, dass sie für den Transport nicht zerlegt werden muss.

## Patentansprüche

1. Vorrichtung (10) zur Wiederbelebung von Patienten mit Herzstillstand mit einer an einem Träger angeordneten Hubvorrichtung (12) für einen auf den Brustkorb des Patienten aufsetzbaren Stempel, der auf- und abbewegbar ist, oder für eine Saugglocke (15) mit Stempel (15.1), wobei der Träger als über den Brustkorb des Patienten stellbares Portal (16) ausgebildet ist und das Portal (16) mindestens in der Breite verstellbar ist, **dadurch gekennzeichnet, dass** am Portal (16) ein Federmechanismus (19), ein Zahnrad-Zahnstangenmechanismus oder ein Seil- oder Kettenzug zur automatischen Ausmittlung der Hubvorrichtung (12) über dem Patienten angeordnet ist.

2. Vorrichtung (10, 39) nach Anspruch 1, **dadurch gekennzeichnet, dass** sie einen Einstellgriff aufweist, um das Portal mindestens in seiner Breite verstellen zu können.

3. Vorrichtung (10, 39) nach Anspruch 1 oder 2, **dadurch gekennzeichnet, dass** die auf den Stempel (15.1) oder die Saugglocke (15) wirkende Bedienkraft insbesondere mittels einer Feder (35) und/oder einer Düse (37) vergrößerbar ist.

4. Vorrichtung (10, 39) nach einem der Ansprüche 1 bis 3, **dadurch gekennzeichnet, dass** die Portalbeine (18) mit nach innen zeigenden Füßen (20) versehen sind, die unter den Brustkorb des Patienten schiebbar sind.

5. Vorrichtung (10, 39) nach einem der Ansprüche 1 bis 4, **dadurch gekennzeichnet, dass** der Querträger (17) des Portals (16) als Teleskoprohr (30) ausgebildet ist.

6. Vorrichtung (10, 39) nach einem der Ansprüche 1 bis 4, **dadurch gekennzeichnet, dass** der linke und der rechte Querträger (40) des Portals (16) versetzt zueinander angeordnet sind.

7. Vorrichtung (10, 39) nach Anspruch 6, **dadurch gekennzeichnet, dass** die Hubvorrichtung (12) mittels eines an den Querträger (40) montierten Seil- oder Kettenzuges über dem Patienten ausmittelbar ist.

8. Vorrichtung (10, 39) nach einem der Ansprüche 1 bis 7, **dadurch gekennzeichnet, dass** die Hubvorrichtung (12) höhenverstellbar am Portal (16) angeordnet ist.

9. Vorrichtung (10, 39) nach einem der Ansprüche 1 bis 8, **dadurch gekennzeichnet, dass** die Hubvorrichtung (12) rein mechanisch über einen Hebel (11), ein Zahnradgetriebe oder andere mechanische Übersetzungen betätigbar ist.

10. Vorrichtung (10, 39) nach einem der Ansprüche 1 bis 9, **dadurch gekennzeichnet, dass** die Hubvorrichtung (12) einen Hubzylinder (13) aufweist, der aus der Umgebung Luft ansaugt, die zur künstlichen Beatmung des Patienten über eine Maske oder einen Tubus an den Patienten abgebbar ist.

11. Vorrichtung (10, 39) nach einem der Ansprüche 1 bis 10, **dadurch gekennzeichnet, dass** sie automatisch zur Herzdruckmassage und Beatmung wechselt.

12. Vorrichtung (10, 39) nach einem der Ansprüche 1 bis 11, **dadurch gekennzeichnet, dass** sie nach dem CPR- und/oder dem ACD-Verfahren betreibbar ist.

13. Vorrichtung (10, 39) nach einem der Ansprüche 1 bis 12, **dadurch gekennzeichnet, dass** der Hubweg für die Herzmassage abhängig von der Brustkorbgröße des Patienten einstellbar ist.

14. Vorrichtung (10, 39) nach Anspruch 13, **dadurch gekennzeichnet, dass** sich der Hubweg für die Herzmassage automatisch und abhängig von der Höhenposition der Hubvorrichtung (12) einstellt.

15. Vorrichtung (10, 39) nach einem der Ansprüche 1 bis 14, **dadurch gekennzeichnet, dass** das Beatmungsvolumen abhängig von der Brustkorbgröße des Patienten einstellbar ist.

16. Vorrichtung (10, 39) nach Anspruch 15, **dadurch gekennzeichnet, dass** das durch den Hubzylinder abgegebene Beatmungsvolumen automatisch abhängig von der Höhenposition der Hubvorrichtung (12) einstellbar ist.

17. Vorrichtung (10, 39) nach einem der Ansprüche 1 bis 16, **dadurch gekennzeichnet, dass** sie eine Skala für den Hubweg und das Beatmungsvolumen aufweist.

18. Vorrichtung (10, 39) nach einem der Ansprüche 1 bis 17, **dadurch gekennzeichnet, dass** sie einen Sauerstoffanschluss für eine Sauerstoffflasche oder für ein dazwischengeschaltetes Sauerstoffreservoir aufweist.

19. Vorrichtung (10, 39) nach einem der Ansprüche 1 bis 16, **dadurch gekennzeichnet, dass** sie zum Transport zerlegbar ist.

20. Vorrichtung (10, 39) nach einem der Ansprüche 1 bis 19, **dadurch gekennzeichnet, dass** sie als reines Beatmungsgerät oder Herzmassagegerät verwendbar ist.

21. Vorrichtung (10, 39) nach einem der Ansprüche 1 bis 20, **dadurch gekennzeichnet, dass** sie wartungsfrei ist.

22. Vorrichtung (10, 39) nach einem der Ansprüche 1 bis 21, **dadurch gekennzeichnet, dass** sie dampfsterilisierbar ist.

23. Vorrichtung (10, 39) nach einem der Ansprüche 1 bis 22, **dadurch gekennzeichnet, dass** der Hubzylinder (13) ein Rückschlagventil aufweist.

24. Vorrichtung (10, 39) nach einem der Ansprüche 1 bis 23, **dadurch gekennzeichnet, dass** diese beim Betreiben der Vorrichtung mit Saugglocke (15) beim Rückhub in eine Höhe, die größer als die Brustkorbhöhe ist, bewegbar ist.

25. Vorrichtung (10, 39) nach einem der Ansprüche 1 bis 24, **dadurch gekennzeichnet, dass** die Dekompressionskraft der Saugglocke (15) durch eine Feder (31) freisetzbar ist, die beim Druckhub vorspannbar ist.

## Claims

1. A device (10) for resuscitating patients suffering from cardiac arrest, comprising a lifting device (12) for a plunger which can be placed on the thorax of the patient and is movable up and down, or for a suction bell (15) with plunger (15.1), which lifting device (12) is arranged on a carrier, the carrier taking the form of a portal (16) which can be placed over the thorax of the patient and the portal (16) being adjustable at least with respect to its width, **characterised in that** a spring mechanism (19), a rack-and-pinion mechanism or a cable winch or chain hoist is arranged on the portal (16) for automatic centring of the lifting device (12) over the patient.

2. A device (10, 39) according to Claim 1, **characterised in that** it has an adjusting handle for adjusting at least the width of the portal.

3. A device (10, 39) according to Claim 1 or 2, **characterised in that** the actuating force acting on the plunger (15.1) or the suction bell (15) can be increased in particular by means of a spring (35) and/or a nozzle (37).

4. A device (10, 39) according to any one of claims 1 to 3, **characterised in that** the legs (18) of the portal are provided with inwardly-oriented feet (20) which can be pushed under the thorax of the patient.

5. A device (10, 39) according to any one of claims 1 to 4, **characterised in that** the crossbeam (17) of the portal (16) takes the form of a telescopic tube (30).

6. A device (10, 39) according to any one of claims 1 to 4, **characterised in that** the left-hand and the right-hand crossbeams (40) of the portal (16) are arranged offset to one another.

7. A device (10, 39) according to Claim 6, **characterised in that** the lifting device (12) can be centred by means of a cable winch or chain hoist mounted on the crossbeam (40).

8. A device (10, 39) according to any one of claims 1 to 7, **characterised in that** the lifting device (12) is arranged height-adjustably on the portal (16).

9. A device (10, 39) according to any one of claims 1 to 8, **characterised in that** the lifting device (12) can be actuated purely mechanically by means of a lever (11), a gear drive or other mechanical transmission.

10. A device (10, 39) according to any one of claims 1 to 9, **characterised in that** the lifting device (12) has a lifting cylinder (13) which sucks in ambient air which can be dispensed to the patient for artificial ventilation of the patient via a mask or a tube.

11. A device (10, 39) according to any one of claims 1 to 10, **characterised in that** it switches automatically to cardiac massage and ventilation.

12. A device (10, 39) according to any one of claims 1 to 11, **characterised in that** it can be operated according to the CPR or the ACD method.

13. A device (10, 39) according to any one of claims 1 to 12 **characterised in that** the travel distance for cardiac massage is adjustable according to the size of the patient's thorax.

14. A device (10, 39) according to Claim 13, **characterised in that** the travel distance for cardiac massage adjusts itself automatically according to the height position of the lifting device (12).

15. A device (10, 39) according to any one of claims 1 to 14, **characterised in that** the respiration volume is adjustable according to the size of the patient's thorax.

16. A device (10, 39) according to Claim 15, **characterised in that** the respiration volume dispensed by means of the lifting cylinder is automatically adjustable according to the height position of the lifting device (12).

17. A device (10, 39) according to any one of claims 1 to 16, **characterised in that** it has a scale for the travel distance and the respiration volume.

18. A device (10, 39) according to any one of claims 1 to 17, **characterised in that** it has an oxygen connection for an oxygen bottle or for an interposed oxygen reservoir.

19. A device (10, 39) according to any one of claims 1 to 18, **characterised in that** it can be dismantled for transportation.

20. A device (10, 39) according to any one of claims 1 to 19, **characterised in that** it is usable purely as a respiration device or as a cardiac massage device.

21. A device (10, 39) according to any one of claims 1 to 20, **characterised in that** it is maintenance-free.

22. A device (10, 39) according to any one of claims 1 to 21, **characterised in that** it is sterilisable by steam.

23. A device (10, 39) according to any one of claims 1 to 22, **characterised in that** the lifting cylinder (13) has a nonreturn valve.

24. A device (10, 39) according to any one of claims 1 to 23, **characterised in that** when the device is operated with the suction bell (15) it can be moved back on the return stroke to a height of which is greater than the height of the thorax.

25. A device (10, 39) according to any one of claims 1 to 24, **characterised in that** the decompression force of the suction bell (15) can be released by a spring (31) which can be pretensioned during the pressure stroke.

## Revendications

1. Dispositif (10) pour réanimer des patients victimes d'arrêt cardiaque comportant un dispositif de levage (12), disposé sur un support, pour un piston à placer sur la cage thoracique du patient, lequel est déplaçable en montée et descente, ou pour une cloche d'aspiration (15) avec piston (15.1), le support étant réalisé comme un portique (16) à placer au-dessus de la cage thoracique du patient, et le portique (16) étant réglable au moins en largeur, **caractérisé en ce que** sur le portique (16) est disposé un mécanisme à ressort (19), un mécanisme à roue dentée et crémaillère ou une commande par câble ou par chaîne pour le positionnement automatique du dispositif de levage (12) au-dessus du patient.

2. Dispositif (10, 39) selon la revendication 1, **caractérisé en ce qu'**il comporte une poignée de réglage pour pouvoir régler le portique au moins dans sa largeur.

3. Dispositif (10, 39) selon la revendication 1 ou 2, **caractérisé en ce que** la force de manoeuvre agissant sur le piston (15.1) ou la cloche d'aspiration (15) peut être augmentée en particulier au moyen d'un ressort (35) et/ou d'une buse (37).

4. Dispositif (10, 39) selon l'une des revendications 1 à 3, **caractérisé en ce que** les jambes (18) du portique sont pourvues de pieds (20) dirigés vers l'intérieur qui peuvent être poussés sous la cage thoracique du patient.

5. Dispositif (10, 39) selon l'une des revendications 1 à 4, **caractérisé en ce que** la traverse (17) du portique (16) est réalisée comme un tube télescopique (30).

6. Dispositif (10, 39) selon l'une des revendications 1 à 4, **caractérisé en ce que** la traverse gauche et la traverse droite (40) du portique (16) sont disposées décalées l'une par rapport à l'autre.

7. Dispositif (10, 39) selon la revendication 6, **caractérisé en ce que** le dispositif de levage (12) peut être centré au-dessus du patient au moyen d'une commande par câble ou par chaîne montée sur la traverse (40).

8. Dispositif (10, 39) selon l'une des revendications 1 à 7, **caractérisé en ce que** le dispositif de levage (12) est disposé réglable en hauteur sur le portique (16).

9. Dispositif (10, 39) selon l'une des revendications 1 à 8, **caractérisé en ce que** le dispositif de levage (12) peut être actionné de manière purement mécanique à travers un levier (11), une transmission à roue dentée ou d'autres transmissions mécaniques.

10. Dispositif (10, 39) selon l'une des revendications 1 à 9, **caractérisé en ce que** le dispositif de levage (12) comporte un cylindre de levage (13) qui aspire, du milieu ambiant, de l'air qui peut être délivré au patient, pour sa respiration artificielle, à travers un masque ou un tube.

11. Dispositif (10, 39) selon l'une des revendications 1 à 10, **caractérisé en ce qu'**il alterne automatiquement entre le massage cardiaque et la respiration.

12. Dispositif (10, 39) selon l'une des revendications 1 à 11, **caractérisé en ce qu'**il peut fonctionner suivant le procédé CPR et/ou ACD.

13. Dispositif (10, 39) selon l'une des revendications 1 à 12, **caractérisé en ce que** la course pour le massage cardiaque peut être réglée en fonction de la taille de la cage thoracique du patient.

14. Dispositif (10, 39) selon la revendication 13, **caractérisé en ce qu'**il règle automatiquement la course pour le massage cardiaque et en fonction de la position en hauteur du dispositif de levage (12).

15. Dispositif (10, 39) selon l'une des revendications 1 à 14, **caractérisé en ce que** le volume de respiration peut être réglé en fonction de la taille de la cage thoracique du patient.

16. Dispositif (10, 39) selon la revendication 15, **caractérisé en ce que** le volume de respiration délivré par le cylindre de levage peut être réglé automatiquement en fonction de la position en hauteur du dispositif de levage (12).

17. Dispositif (10, 39) selon l'une des revendications 1 à 16, **caractérisé en ce qu'**il comporte une échelle pour la distance de course et le volume de respiration.

18. Dispositif (10, 39) selon l'une des revendications 1 à 17, **caractérisé en ce qu'**il comporte un raccord d'oxygène pour une bouteille d'oxygène ou pour un réservoir d'oxygène intercalé.

19. Dispositif (10, 39) selon l'une des revendications 1 à 18, **caractérisé en ce qu'**il peut être démonté pour le transport.

20. Dispositif (10, 39) selon l'une des revendications 1 à 19, **caractérisé en ce qu'**il peut être utilisé uniquement comme un appareil de respiration ou comme un appareil de massage cardiaque.

21. Dispositif (10, 39) selon l'une des revendications 1 à 20, **caractérisé en ce qu'**il ne nécessite pas d'entretien.

22. Dispositif (10, 39) selon l'une des revendications 1 à 21, **caractérisé en ce qu'**il peut être stérilisé à la vapeur.

23. Dispositif (10, 39) selon l'une des revendications 1 à 22, **caractérisé en ce que** le cylindre de levage (13) comporte un clapet de antiretour.

24. Dispositif (10, 39) selon l'une des revendications 1 à 23, **caractérisé en ce que** pendant le fonctionnement du dispositif avec cloche d'aspiration (15), il peut être déplacé, lors de la course de retour, à une hauteur qui est supérieure à la hauteur de la cage thoracique.

25. Dispositif (10, 39) selon l'une des revendications 1 à 24, **caractérisé en ce que** la force de décompression de la cloche d'aspiration (15) peut être libérée par un ressort (31) qui peut être précontraint lors de la course de compression.
